# EUROPÄISCHE PATENTSCHRIFT

(11) **EP 1 036 760 B1**
(45) Veröffentlichungstag und Bekanntmachung des Hinweises auf die Patenterteilung: **01.09.2004**
(21) Anmeldenummer: 00103129.3
(22) Anmeldetag: 16.02.2000
(51) Int. Cl.: C01B 17/66, C07C 313/04

(54) **Zusammensetzung, die mindestens ein Sulfinsäurederivat und Kaliumcarbonat enthält**
Composition containing at least a sulfinic acid derivative and potassium carbonate
Composition contenant au moins un dérivé d'acide sulfinique et du carbonate de potassium

(30) Priorität: 15.03.1999 DE 19911357
(43) Veröffentlichungstag der Anmeldung: 20.09.2000
(73) Patentinhaber: BASF AKTIENGESELLSCHAFT, 67056 Ludwigshafen (DE)
(72) Erfinder: Beckmann, Eberhard, Dr., 67435 Neustadt (DE); Krüger, Rudolf, Dr., 67273 Weisenheim (DE)

(56) Entgegenhaltungen:
- EP-A- 0 153 671
- DATABASE CHEMABS [Online] CHEMICAL ABSTRACTS SERVICE, COLUMBUS, OHIO, US; STN, CAPLUS accession no. 1985:222115, XP002141543 & SU 1 143 786 A (ALL-UNION CORRESPONDENCE INSTITUTE OF THE TEXTILE AND LIGHT INDUSTRY) 7. März 1985 (1985-03-07)
- DATABASE CHEMABS [Online] CHEMICAL ABSTRACTS SERVICE, COLUMBUS, OHIO, US; CAPLUS, STN accession no. 1977:57568, XP002141544 -& JP 51 110497 A (MITSUBISHI GAS CHEMICAL CO INC) 30. September 1976 (1976-09-30)

## Beschreibung

Die vorliegende Erfindung betrifft Sulfinsäurederivate enthaltende Zusammensetzungen. Sie betrifft insbesondere solche Zusammensetzungen, die Sulfinsäurederivate mit reduzierenden Eigenschaften enthalten, und in denen das Sulrinsaurederivat durch Zugabe von Stabilisatoren gegen Zersetzung stabilisiert ist.

Sulfinsäurederivate sind Derivate der Sulfinsäure mit der allgemeinen Formel

R-S(O)-O⁻ M⁺.

Der Substituent R bedeutet einen organischen Rest, beispielsweise einen substituierten oder unsubstituierten Alkyl- oder Arylrest oder einen anorganischen Rest, beispielsweise einen substituierten oder unsubsticuierten Sulfino- oder Sulfinatorest. Der Substituent M⁺ steht für ein Kation, beispielsweise ein Proton, ein einfach positiv geladenes Metallkation oder das zum Ausgleich der elektrischen Ladungen erforderliche Äquivalent eines mehrfach positiv geladenen Metallkations.

Sulfinsäurederivate weisen reduzierende Eigenschaften auf und sind technisch wichtige Hilfsmittel, vor allem in der Textil- und Papierindustrie, beispielsweise zur Reduktion von Farbstoffen in der Küpenfärberei oder zur Bleiche von holzschliffhaltigem Papier.

Sulfinsäurederivate sind im allgemeinen jedoch nicht lagerstabil. Sie sind daher trocken, gut verschlossen und kühl aufzubewahren, zersetzen sich aber dennoch bei Lagerung, ein Prozeß, der unter ungünstigen Umständen bis zur Selbstentzündung der gelagerten Ware führen kann, was Sulfinsäurederivate zum Gefahrgut machen kann. Abgesehen von dieser Begleiterscheinung führt ihre Zersetzung zu einer steten Abnahme des Gehalts der gelagerten Ware an Sulfinsäurederivaten und zur Entwicklung eines unangenehmen Geruchs nach Schwefeldioxid und/oder Schwefelwasserstoff, der sich z. B. in Färbeprozessen auch auf die gefärbten Stoffe und damit auf die daraus hergestellte Kleidung überträgt. Dieser Geruch kann zwar durch Parfümierung übertönt werden, dies erfordert jedoch Mehraufwand und löst weder das zugrundeliegende Problem noch die anderen mit der mangelnden Lagerstabilität verbundenen Probleme, beispielsweise den steten Wertverlust gelagerter Ware, da ein abnehmender Gehalt an Sulfinsäurederivac in der Zusammensetzung einen steten Mehrbedarf an Zusammensetzung zur Erzielung der gleichen Wirkung bedeutet. Ein weiterer mit der mangelnden Lagerstabilität verbundener Nachteil ist die Verklebungsneigung, die die Handhabung des Produkts, beispielsweise sein Rieselverhalten bei der Entnahme aus Bunkern oder seine Transportierbarkeit in Förderschnecken beeinträchtigt. Die Lagerstabilität der technisch meist verwendeten Natrium-Sulfinsäuresalze ist zudem geringer als die der seltener verwendeten Zink-Sulfinsäuresalze, die aufgrund der Toxizität von Zinkionen für Wasserlebewesen nicht für jeden Anwendungsfall geeignet sind.

Sulfinsäurederivate werden daher üblicherweise in Form von Zusammensetzungen gehandelt und verwendet, in denen die Sulfinsäurederivate durch Zusatz von Stabilisatoren gegen Zersetzung stabilisiert sind. So lehrt beispielsweise JP-A-1970/26 610 (Derwent Abstract No. 61104R-E) die Zumischung von 1-50 Teilen wasserfreiem Natriumbisulfit zu 100 Teilen Natriumdithionit. JP-A-51 110 497 (Derwent Abstract No. 85930X/46) lehrt stabilisierte Dithionitzubereitungen, die Alkalimetallcarbonat und Alkalimetallbicarbonat oder Alkalimetallbenzoat enthalten. In DE-A-20 31 820 wird die Stabilisierung von festen Natriumdithionitpartikeln durch Überzüge mit oxipropylierter Cellulose oder Stärke offengelegt. US-A-3,054,658 lehrt die Stabilisierung von Natriumdithionit durch Zumischung eines Natrium- oder Kaliumsalzes einer C₁-bis C₁₀-Fettsäure oder der Benzoesäure. DE-A-21 07 959 lehrt die Stabilisierung von Natriumdithionit durch Zumischung von kalzinierter Soda und diethylentriaminpentaessigsaurem Natrium. US-A-5,296,210 offenbart die Stabilisierung von Natriumdithionit durch oxidische, wasserabsorbierende Verbindungen oder Verbindungsgemischen mit alkalischen Eigenschaften. Explizit genannt werden CaO, BaO, CaSO₄/Na₂CO₃, SiO₂/Na₂CO₃, Al₂O₃/Na₂CO₃ und CaO/BaO. Diese Substanzen weisen jedoch den Nachteil der Unlöslichkeit in den üblichen gebrauchsfertigen, insbesondere wäßrigen Lösungen von Sulfinsäurederivaten auf, was deren technische Handhabung und Verwendung erschwert. Die deutsche Patentanmeldung Nr. 19905395.2 (Anmeldetag 10.02.1999) lehrt Zusammensetzungen, die mindestens ein Sulfinsäurederivat und Magnesiumsulfat enthalten. Diese Zusammensetzungen zeigen zwar wesentlich verlangsamte Zersetzung des Sulfinsäurederivats und wesentlich verbesserte Geruchseigenschaften, die Erfahrung zeigt jedoch, daß sie in manchen Anwendungsbereichen in den dort üblicherweise verwendeten Lösungen zur Bildung unerwünschter oder sogar störender Trübungen oder Niederschläge führen können.

Es bestand daher die Aufgabe, eine weitere Sulfinsäurederivate enthaltende Zusammensetzung zu finden, in der die Sulfinsäurederivate einerseits besser gegen Zersetzung stabilisiert sind als in den meisten bekannten Zusammensetzungen und andererseits die Bildung von Trübungen oder Niederschlägen bei der Anwendung dieser Zusammensetzung möglichst vermieden wird.

Dementsprechend wurde eine feste Zusammensetzung gefunden, die mindestens ein Sulfinsäurederivat und Kaliumcarbonat enthält. Die erfindungsgemäße Zusammensetzung zeigt gegenüber den bekannten Zusammensetzungen eine deutliche Verbesserung in der Stabilität, insbesondere eine geringere Abnahme des Gehalts an Sulfonsäurederivat und eine geringere Entwicklung unangenehmer Gerüche, und ein verringertes Auftreten von Trübungen oder Niederschlägen in Lösungen, die diese Zusammensetzung enthalten.

Die erfindungsgemäße Zusammensetzung ist eine feste Zusammensetzung und in bevorzugter Form eine feste, rieselfähige Zusammensetzung, aus der beispielsweise durch Auflösen in einem Lösungsmittel wie Wasser die technisch üblicherweise verwendete Lösung des Sulfinsäurederivats oder der Sulfinsäurederivate hergestellt wird.

Die erfindungsgemäße Zusammensetzung enthält mindestens ein Sulfinsäurederivat, dabei kann jedes bekannte Sulfinsäurederivat verwendet werden.

Sulfinsäurederivate werden durch die allgemeine Formel

R-S(O)-O⁻ M⁺

beschrieben.

Der Substituent R bedeutet einen organischen Rest, beispielsweise einen substituierten oder unsubstituierten Alkyl- oder Arylrest oder einen anorganischen Rest, beispielsweise einen substituierten oder unsubstituierten Sulfino- oder Sulfinatorest.

Als Alkylreste sind geradkettige oder verzweigte C₁ bis C₁₀-Alkylreste geeignet, die auch substituiert sein können. Bevorzugt ist die Verwendung von C₁-bis C₄-Alkylresten wie Methyl- Ethyl- n-Propyl-, i-Propyl-, n-Butyl-, sec.-Butyl-, iso-Butyl- oder tert.-Butylresten, besonders bevorzugt ist die Verwendung eines Methylrests. Statt Wasserstoffsubstituenten können in diesen Resten andere Substituenten verwendet werden, beispielsweise Halogene wie Fluor, Chlor, Brom oder Iod, sauerstoffhaltige Substituenten wie Oxi-, Alkoxi- und/oder Hydroxireste und/oder stickstoffhaltige Substituenten wie Amino- und/oder Iminoreste. Bevorzugterweise werden Hydroxi- oder Amino- und Iminoreste verwendet. Besonders bevorzugt ist die Verwendung eines 1-Hydroxialkylrests, insbesondere eines 1-Hydroxiethyl- oder Hydroximethylrests (R = CH₃-CH(OH)- oder HO-CH₂-) oder eines Aminoiminomethylrests (R = H₂N-(HN)C-)oder von Kondensationsprodukten der Hydroxialkylreste mit Ammoniak (R = H₂N-CHR'-, M^{+ -}O-(O)S-CHR'-NH-CHR'- oder [M^{+ -}O-(O)S-CHR']₂N-CHR'-, wobei M⁺ die unten definierte Bedeutung hat und R' für Wasserstoff oder einen Alkylrest steht, insbesondere für Methyl. Treten M⁺ und R' in einer Verbindung mehrfach auf, können sie jeweils verschiedene Substituenten bedeuten.)

Als Arylreste sind aromatische Reste mit mindestens 6 Kohlenstoffatomen geeignet, die auch substituiert sein können. Bevorzugt ist die Verwendung von substituierten oder unsubstituierten Phenylresten. Statt Wasserstoffsubstituenten können in diesen Resten andere Substituenten verwendet werden, beispielsweise Halogene wie Fluor, Chlor, Brom oder Iod, sauerstoffhaltige Substituenten wie Alkoxi- und/oder Hydroxireste und/oder stickstoffhaltige Substituenten wie Alkylamino- oder Aminoreste.

Als anorganischer Rest sind alle Reste geeignet, die die Stabilität der Sulfinsäureeinheit nicht beeinträchtigen. Bevorzugterweise werden schwefelhaltige Reste verwendet, und in besonders bevorzugter Weise wird ein Sulfino- oder Sulfinatorest verwendet (R = X^{+ -}O-(O)S-).

Als Substituent M⁺ der Sulfinsäurederivate wird ein Kation verwendet, etwa ein Proton, ein einfach positiv geladenes Metallkation oder das zum Ausgleich der elektrischen Ladungen erforderliche Äquivalent eines mehrfach positiv geladenen Metallkations.

Als einfach positiv geladenes Kation kann beispielsweise ein Kation eines Alkalimetalls wie Lithium, Natrium, Kalium, Rubidium oder Cäsium verwendet werden, unter diesen sind Natrium oder Kalium bevorzugt, Natrium ist besonders bevorzugt. Als mehrfach positiv geladenes Kation kann beispielsweise ein Kation eines Erdalkalimetalls wie Magnesium, Calcium, Strontium oder Barium oder eines anderen zweiwertigen Metalls wie Zink verwendet werden, unter diesen sind Magnesium, Calcium oder Zink bevorzugt, Zink ist besonders bevorzugt. Es können auch Gemische von ein- und/oder mehrfach positiv geladenen Kationen verwendet werden, solange insgesamt die Ladungsneutralität gewahrt ist.

In bevorzugter Form enthält die erfindungsgemäße Zusammensetzung mindestens ein aus der Gruppe:
Zinkdithionit ZnS₂O₄,
Natriumdithionit Na₂S₂O₄,
Zink-1-hydroxiethansulfinat (H₃C-CH(OH)-SO₂)₂Zn,
Natrium-1-hydroxiethansulfinat H₃C-CH(OH)-SO₂Na,
Zink-1-aminoethansulfinat (H₃C-CH(NH₂)-SO₂)₂Zn,
Natrium-1-aminoethansulfinat H₃C-CH(NH₂)-SO₂Na,
Zink-1-iminoethansulfinat HN[-CH(CH₃)-SO₂]₂Zn,
Natrium-1-iminoethansulfinat HN[-CH(CH₃)-SO₂Na]₂

Zink-1-nitriloethansulfinat [N[-CH(CH₃)-SO₂]₃]₂Zn₃,
Natrium-1-nitriloethansulfinat N[-CH(CH₃)-SO₂Na]3,
Zinkhydroximethansulfinat (H₂C(OH)-SO₂)₂Zn,
Natriumhydroximethansulfinat H₂C(OH)-SO₂Na,
Zinkaminomethansulfinat (H₂C(NH₂)-SO₂)₂Zn,
Natriumaminomethansulfinat H₂C(NH₂)-SO₂Na,
Zinkiminomethansulfinat HN[-CH₂-SO₂]₂Zn,
Natriumiminomethansulfinat HN[-CH₂-SO₂Na]₂,
Zinknitrilomethansulfinat [N[-CH₂-SO₂]₃]₂Zn₃,
Natriumnitrilomethansulfinat N[-CH₂-SO₂Na]3 und
Aminoiminomethansulfinsäure H₂N-(HN)C-SO₂H
gewähltes Sulfinsäurederivat. In besonders bevorzugter Form enthält sie Zinkdithionit und/oder Natriumdithionit und in ganz besonders bevorzugter Form Natriumdithionit.

Dithionite (R = M^{+ -}O-(O)S-), oftmals auch als Hypodisulfite oder Hydrosulfite bezeichnet, werden üblicherweise durch Reduktion von schwefliger Säure oder Hydrogensulfit mittels starker Reduktionsmittel hergestellt. Technisch wird diese Reduktion meist mit Zinkstaub, Natriumamalgam oder Natriumformiat als Reduktionsmittel durchgeführt, wobei entsprechend Zinkdithionit oder Natriumdithionit entsteht. Die mit den einzelnen genannten Verfahren gewonnenen technischen Dithionite werden häufig auch als "Zinkstaubware", "Amalgamware" oder "Formiatware", respektive, bezeichnet. Hydroximethylsulfinate (R = HO-CH₂-) werden üblicherweise durch Reduktion des entsprechenden Sulfonylchlorids R-SO₂Cl mittels Zinkstaub, Natriumamalgam oder Natriumformiat als Reduktionsmittel hergestellt, wobei das entsprechende Zink- oder Natriumsalz entsteht. Diese Produkte werden mit Ammoniak zu den entsprechenden Amino-, Imino- oder Nitriloverbindungen umgesetzt. Aminoiminomethansulfinsäure (R = H₂N(HN)C-, M⁺ = H⁺), auch als Formamidinsulfinsäure oder als Thioharnstoffdioxid bezeichnet, da sie im festen Zustand vorwiegend in der tautomeren Struktur O₂S=C(NH₂)₂ vorliegt, wird üblicherweise durch Oxidation von Thioharnstoff mit Wasserstoffperoxid hergestellt.

Ein wesentlicher Unterschied der Reduktion der entsprechenden Ausgangsverbindung mittels Formiaten zu den anderen gängigen Verfahren zur Herstellung von Sulfinsäurederivaten ist, daß die Fällung im sauren pH-Bereich durchgeführt wird. In einer bevorzugten Ausführungsform der Erfindung enthält die Zusammensetzung minäestens ein Sulfinsäurederivat, das durch Reduktion der entsprechenden Ausgangsverbindung im sauren pH-Bereich, insbesondere durch Reduktion der entsprechenden Ausgangsverbindung mittels eines Formiats, speziell Natriumformiat, hergestellt wurde.

Technische Sulfinsäurederivate fallen meist nicht in reiner Form an, sondern enthalten einen gewissen Anteil Nebenkomponenten, etwa unumgesetzte Ausgangsverbindungen, Nebenprodukte der Reduktion, Zersetzungsprodukte der Sulfinsäurederivate und/oder bei ihrer Herstellung zugesetzte Hilfsstoffe, beispielsweise Natriumcarbonat oder Hexamethylentetramin. Der Anteil solcher Nebenkomponenten liegt im allgemeinen zwischen etwa 5 Gew.-% und 20 Gew.-%, bezogen auf die Gesamtmenge des technischen Produkts.

Die erfindungsgemäße Zusammensetzung enthält ferner Kaliumcarbonat. In bevorzugter Form enthält sie wasserfreies Kaliumcarbonat.

Der Gehalt einer solchen frisch hergestellten Zusammensetzung an Sulfinsäurederivaten und Kaliumcarbonat kann praktisch beliebig variiert werden, wobei im Einzelfall durch Routineexperimente ein Kompromiss zwischen dem aus wirtschaftlichen Gründen erstrebten möglichst hohen Gehalt an Sulfinsäurederivaten und einer für die geforderte Lagerstabilität ausreichend hohen Kaliumcarbonatmenge gefunden werden muß.

Die erfindungsgemäße Zusammensetzung kann aus mindestens einem Sulfinsäurederivat, beispielsweise Zinkdithionit, Natriumdithionit, Zinkhydroximethansulfinat, Natriumhydroximethansulfinat, deren Kondensationsprodukten mit Ammoniak, und/oder Aminoiminomethansulfinsäure und Kaliumcarbonat bestehen. In diesem Fall liegt der Gehalt der frisch hergestellten Zusammensetzung an Kaliumcarbonat im allgemeinen zwischen 0,1 und 15 Gew.-%, in bevorzugter Form zwischen 0,5 und 5 Gew.-% und in besonders bevorzugter Form zwischen 1 und 3 Gew.-%. In ganz besonders bevorzugter Form wird ein Kaliumcarbonatgehalt unterhalb von 2 Gew.-% eingestellt, beispielsweise von 1 Gew.-%. Diese Gew.-%-Werte sind jeweils bezogen auf die gesamte Zusammensetzung und berechnet als wasserfreies Kaliumcarbonat.

Die erfindungsgemäße Zusammensetzung kann aber auch neben dem Sulfinsäurederivat oder den Sulfinsäurederivaten und Kaliumcarbonat beliebige weitere Bestandteile enthalten. Sie kann insbesondere neben den bei der industriellen Herstellung von Sulfinsäurederivaten üblicherweise anfallenden und daher in den technischen Produkten enthaltenen Nebenkomponenten oder unumgesetzten Ausgangsstoffen die bereits aus dem Stand der Technik bekannten Stabilisatoren für Sulfinsäurederivate und/oder alle anderen bekannten Zusätze zu handelsüblichen Zubereitungen von Sulfinsäurederivaten enthalten. Sie kann beispielsweise zusätzliche Hilfsmittel wie etwa Aktivatoren, Komplexbildner wie etwa Nitrilotriessigsäuresalze und/oder Ethylendiamintetraessigsäuresalze, optische Aufheller, Duftstoffe und/oder oberflächenaktive Substanzen wie etwa Tenside und/oder Dispergiermittel enthalten. Beispiele für solche zusätzlichen Bestandteile der erfindungsgemäßen Zusammensetzung sind Alkalisalze wie Natrium- und/oder Kaliumcarbonat, -hydrogencarbonat, -sulfit, -hydrogensulfit, -disulfit, -sulfat, -mono-, -di-, -tri- und/oder -polyphosphat, -phosphonat und/oder -carboxilat, Tenside und Kohlenhydrate wie Stärke, Cellulose, und/oder Zucker, beispielsweise Glucose, Fructose, Maltose oder Saccharaso, die auch oxialkyliert sein können. Ob solche zusätzlichen Bestandteile, insbesondere die genannten Metallsalze, zugesetzt werden und gegebenenfalls ihre Menge ist anhand des konkreten Anwendungsfalls durch Routineexperimente zu optimieren. Zusätzliche Bestandteile, die in der zugesetzten Menge zu Trübungen oder Niederschlägen führen, sowie für den gewünschten technischen Effekt unnötige Bestandteile werden nicht oder nur in verringerter Menge zugesetzt. In bevorzugter Form enthält die Zusammensetzung kein Magnesiumsulfat, neben Kaliumcarbonat und Natriumcarbonat keine weiteren Carbonate, vorzugsweise auch kein Natriumcarbonat, und/oder keine Bicarbonate. Weiterhin enthält die Zusammensetzung in bevorzugter Form Zucker und/oder Komplexbildner. Wenn zugesetzt, sind Zucker und/oder Komplexbildner jeweils in einer Menge von im allgemeinen mindestens 0,2 Gew.-%, bevorzugterweise mindestens 1 Gew.-% und in besonders bevorzugter Weise mindestens 2 Gew.-%, sowie im allgemeinen höchstens 20 Gew.-%, bevorzugterweise höchstens 15 Gew.-% und in besonders bevorzugter Weise höchstens 10 Gew.-%, jeweils bezogen auf die gesamte Zusammensetzung, enthalten.

Im allgemeinen enthält eine solche, frisch hergestellte erfindungsgemäße Zusammensetzung insgesamt 15 bis 90 Gew.-% des Sulfinsäurederivats oder der Sulfinsäurederivate, vorzugsweise 30 bis 85 Gew.-% und in besonders bevorzugter Weise 40 bis 80 Gew.-%, jeweils bezogen auf die gesamte Zusammensetzung. Ihr Kaliumcarbonat-Gehalt, berechnet als wasserfreies Kaliumcarbonat, beträgt im allgemeinen mindestens 0,01 Gew.-%, vorzugsweise mindestens 0,2 Gew.-% und in besonders bevorzugter Weise mindestens 0,5 Gew.-% sowie im allgemeinen höchstens 10 Gew.-%, vorzugsweise höchstens 5 Gew.-% und in besonders bevorzugter Weise höchstens 3 Gew.-%, jeweils bezogen auf die gesamte Zusammensetzung, wobei die sonstigen Bestandteile der erfindungsgemäßen Zusammensetzung die Gewichtsanteile von Sulfinsäurederivaten und Kaliumcarbonat zu 100 Gew.-% ergänzen.

Hergestellt wird die erfindungsgemäße Zusammensetzung durch Zumischen von Kaliumcarbonat während oder nach der Herstellung des Sulfinsäurederivats oder der Sulfinsäurederivate oder dessen oder deren handelsüblichen Zusammensetzungen. Art und Zeitpunkt der Zugabe von Kaliumcarbonat sind im allgemeinen unkritisch und können auf jede bekannte Weise erfolgen. Beispielsweise kann festes Kaliumcarbonat oder eine Kaliumcarbonat enthaltende Lösung oder Suspension in einem geeigneten Lösungs- oder Suspensionsmittel oder einem Gemisch solcher Lösungs- oder Suspensionsmittel in die Reaktionsmischung zur Herstellung eines Sulfinsäurederivats oder an jeder Stufe zugegeben werden. Geeignete Lösungs- oder Suspensionsmittel sind beispielsweise Wasser, Ether wie etwa Diethylether, Di-n-propylether, Di-iso-propylether, Di-n-butylether, Methyl-tert.-butylether, Ethyl-tert.-butylether und/oder Kohlenwasserstoffe wie Pentan, Hexan, Benzol, Toluol, oder Xylol.

Statt Kaliumcarbonat selbst können auch andere Substanzen zugegeben werden, deren Zugabe zum Vorhandensein von Kaliumcarbonat in der fertigen Zusammensetzung führt. So können von Kaliumcarbonat verschiedene Kaliumsalze und Quellen von Carbonationen zugegeben werden, beispielsweise Kaliumhalogenide wie Kaliumchlorid und/oder -bromid, Kaliumsulfat, Kaliumnitrat, Kaliumsulfit, Alkalicarbonate wie Natriumcarbonat und/oder Ammoniumcarbonat.

Bevorzugterweise wird festes Kaliumcarbonat und in besonders bevorzugter Form wasserfreies festes Kaliumcarbonat beim üblichen Abmischvorgang zur Herstellung handelsüblicher Zusammensetzungen von Sulfinsäurederivaten, bei dem das technische Sulfinsäurederivat oder die technischen Sulfinsäurederivate mit bekannten Zusatzstoffen vermischt werden, zugesetzt. Das Kaliumcarbonat wird in den in solchen Zusammensetzungen üblichen Korngrößen von festen Zusatzstoffen zugesetzt, in diesem Rahmen ist die Korngröße des Kaliumcarbonats praktisch beliebig variierbar. Bei feinteiligerem Kaliumcarbonat tritt üblicherweise eine bessere Stabilisierungswirkung auf, allerdings wird durch die bei sehr feinteiligem Gut vermehrte Staubbildung die Handhabung des Produkts erschwert, so daß unter Umständen für einzelne Anwendungsfälle eine optimale Korngröße des zugesetzten Kaliumcarbonats festzulegen ist. Im allgemeinen beträgt die mittlere Korngröße (d. h., 50 Gew.-% des Kaliumcarbonats werden von einem Sieb der entsprechenden Maschenweite zurückgehalten) von 0,005 bis 1,0 Millimeter, meist von 0,02 bis 0,5 Millimeter.

Die erfindungsgemäßen Zusammensetzungen können länger oder bei höherer Temperatur (beispielsweise bei Färbebetrieben in tropischen Ländern) gelagert werden als die bekannten Zusammensetzungen oder weisen bei vergleichbar langer Lagerungsdauer eine geringere Bildung unangenehmer Gerüche, eine geringere Abnahme des Gehalts an Sulfinsäurederivat oder Sulfinsäurederivaten, und/oder eine geringere Neigung zur Selbstentzündung auf. Gleichermaßen ist die Verklebungsneigung der erfindungsgemäßen Zusammensetzung gegenüber handelsüblichen Zusammensetzungen reduziert. Sie können beispielsweise problemlos mittels Schnecken dosiert werden, ohne daß Anbackungen entstehen. Weiterhin führen die erfindungsgemäßen Zusammensetzungen in den meisten Anwendungsgebieten nicht zu Trübungen oder Niederschlägen.

### Beispiele

Es wurden 17 verschiedene Zusammensetzungen von Sulfinsäurederivaten hergestellt, deren Zusammensetzungen in Tabelle 1 angegeben sind. Ihr Gehalt an Dithionit wurde iodometrisch bestimmt. Diese Zusammensetzungen wurden jeweils eine Woche bei 50 °C gelagert (Vergleichsversuche V1 bis V17), anschließend wurde wiederum der Gehalt an Dithionit bestimmt und der Geruchseindruck sensorisch bewertet. In den Zusammensetzungen der Vergleichsversuche 11 und 12 war kein Dithionit enthalten, sondern Natrium- oder Zinkhydroximethansulfinat, die Bestimmung des Dithionitgehalts entfiel daher.

Parallel zu diesen Vergleichsversuchen wurden 17 weitere Zusammensetzungen hergestellt, die sich von denjenigen der Vergleichsversuche durch den Ersatz eines Anteils an Dithionit oder Hydroximethansulfinat durch Kaliumcarbonat unterschieden, wie in Tabelle 2 aufgeführt. Diese Zusammensetzungen wurden ebenfalls eine Woche bei 50 °C gelagert (Versuche 1 bis 17), anschließend wurde ebenfalls der Gehalt an Dithionit (Ausnahme: die dithionitfreien Zusammensetzungen der Versuche 11 und 12) bestimmt und der Geruchseindruck sensorisch bewertet.

### Erläuterungen zu den Tabellen 1 und 2

In den Tabellen 1 und 2 sind als Vergleichsbeispiele V1 bis V17 und erfindungsgemäße Beispiele 1 bis 17 Sulfinsäurederivate enthaltende Zusammensetzungen mit ihren Stabilitäts- und Geruchseigenschaften aufgeführt.

Sämtliche Zahlenangaben sind in Gew.-%, jeweils bezogen auf die gesamte Zusammensetzung.

Die verwendeten Markennamen der BASF Aktiengesellschaft, Ludwigshafen, Deutschland, für manche Zusammensetzungsbestandteile bezeichnen handelsübliche Zubereitungen der folgenden Chemikalien:

| | |
|---|---|
| Rongalit® C | Natriumhydroximethansulfinat, |
| Decrolin® | Zinkhydroximethansulfinat, |
| Trilon® A | Nitrilotriessigsäure und ihre (Natrium-) Salze, |
| Trilon® B | Ethylendiamintetraessigsäure und ihre (Natrium-) Salze. |

Dequest® ist eine Marke der Monsanto Company, U.S.A., für ihre Phosphonate. Diese sind gängige Handelswaren.

Bei der Geruchsprüfung bedeutet:
- --: stark unangenehm, für technische Anwendungen nicht tolerierbar;
- -: unangenehm, für technische Anwendungen nur mit Maskierung, beispielsweise durch Parfümierung, tolerierbar;
- +: wenig unangenehm, für technische Anwendungen tolerierbar;
- ++: neutral, nicht unangenehm.

### Ergebnis

In allen Fällen ergab sich für die erfindungsgemäßen Zusammensetzungen der Versuche 1 bis 17 jeweils im Vergleich mit den nicht erfindungsgemäßen Zusammensetzungen der Vergleichsversuche V1 bis V17 eine deutliche Verbesserung der Geruchseigenschaften des gelagerten Produkts, und in den meisten Fällen eine deutlich geringere meßbare Abnahme des ursprünglichen Dithionitgehalts, insbesondere dort, wo die Zusammensetzung des entsprechenden Vergleichsbeispiels eine vergleichsweise schnellere Abnahme des Dithionitgehalts zeigt.

Der Zusatz von Kaliumcarbonat zu den bekannten, Sulfinsäurederivate enthaltenden Zusammensetzungen führt also zu einer geringeren Geruchsbelästigung und zu einer besseren Stabilisierung der verwendeten Sulfinsäurederivate und ist damit eine bequeme und wohlfeile Methode zur Stabilisierung von Sulfinsäurederivate enthaltenden Zusammensetzungen.

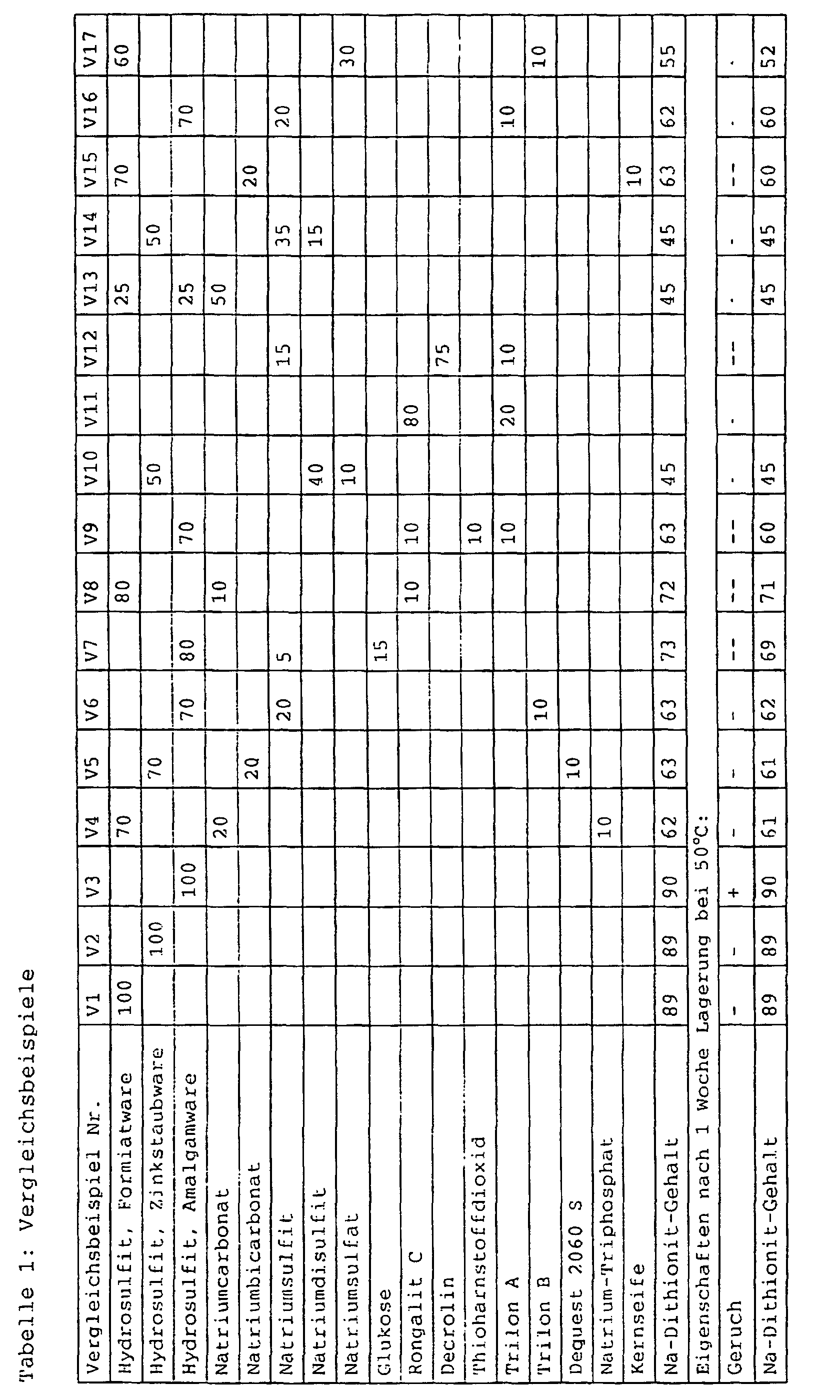

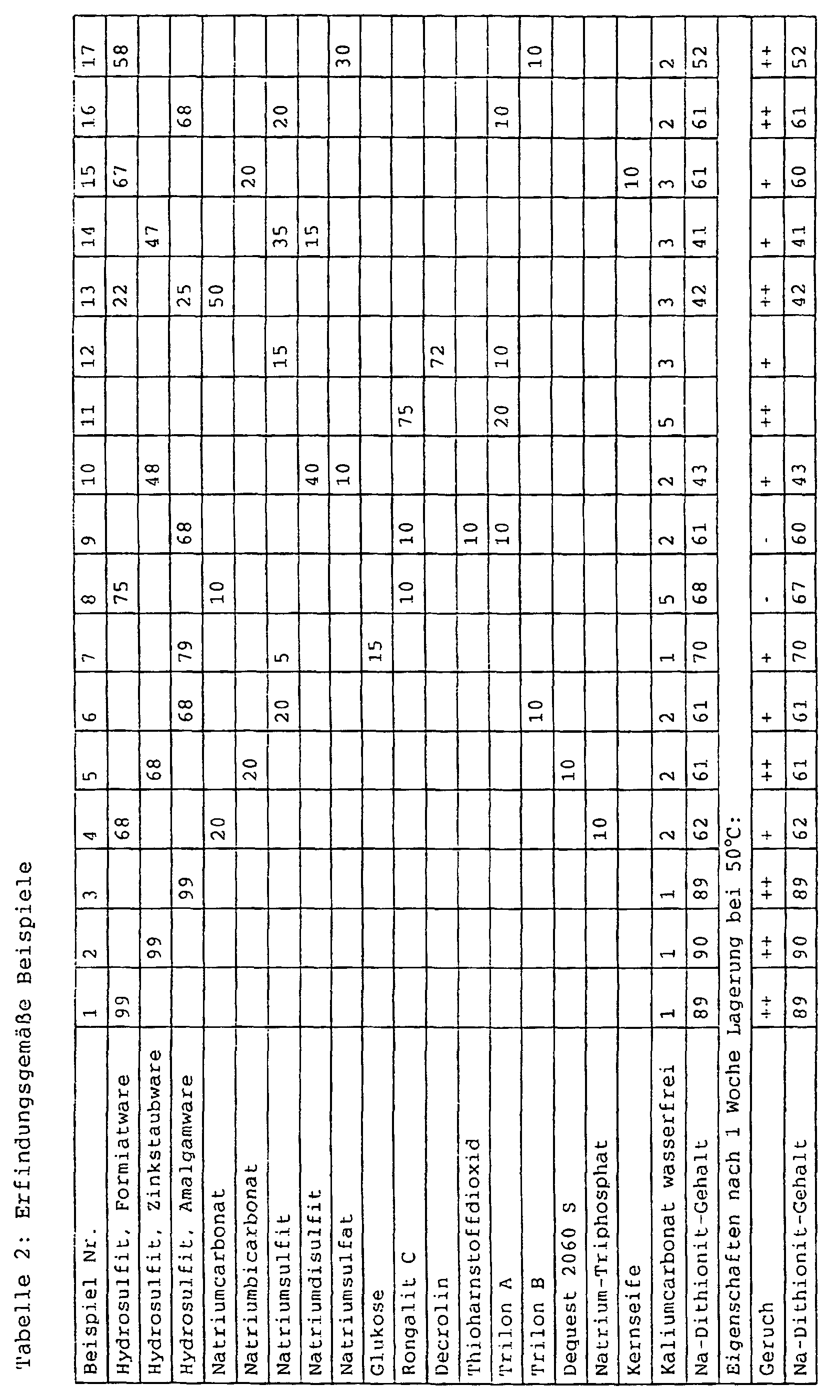

## Patentansprüche

1. Feste Zusammensetzung, die mindestens ein Sulfinsäurederivat und Kaliumcarbonat enthält.

2. Zusammensetzung nach Anspruch 1, die mindestens ein aus der Gruppe Zinkdithionit, Natriumdithionit, Zink-1-hydroxiethansulfinat, Natrium-1-hydroxiethansulfinat, Zink-1-aminoethansulfinat, Natrium-1-aminoethansulfinat, Zink-1-iminoethansulfinat, Natrium-1-iminoethansulfinat, Zink-1-nitriloethansulfinat, Natrium-1-nitriloethansulfinat, Zinkhydroximethansulfinat, Natriumhydroximethansulfinat, Zinkaminomethansulfinat, Natriumaminomethansulfinat, Zinkiminomethansulfinat, Natriumiminomethansulfinat, Zinknitrilomethansulfinat, Natriumnitrilomethansulfinat und Aminoiminomethansulfinsäure gewähltes Sulfinsäurederivat enthält.

3. Zusammensetzung nach Anspruch 2, die Zinkdithionit und/oder Natriumdithionit enthält.

4. Zusammensetzung nach Anspruch 3, die Natriumdithionit enthält.

5. Zusammensetzung nach den Ansprüchen 1 bis 4, die insgesamt 15 bis 90 Gew.-% Sulfinsäurederivat oder -derivate, bezogen auf die gesamte Zusammensetzung, enthält.

6. Zusammensetzung nach den Ansprüchen 1 bis 5, die 0,01 bis 10 Gew.-% Kaliumcarbonat, bezogen auf die gesamte Zusammensetzung, enthält.

## Claims

1. A solid composition which contains at least one sulfinic acid derivative and potassium carbonate.

2. A composition as claimed in claim 1, which contains at least one sulfinic acid derivative selected from the group consisting of zinc dithionite, sodium dithionite, zinc 1-hydroxyethanesulfinate, sodium 1-hydroxyethanesulfinate, zinc 1-aminoethanesulfinate, sodium 1-aminoethanesulfinate, zinc 1-iminoethanesulfinate, sodium 1-iminoethanesulfinate, zinc 1-nitriloethanesulfinate, sodium 1-nitriloethanesulfinate, zinc hydroxymethanesulfinate, sodium hydroxymethanesulfinate, zinc aminomethanesulfinate, sodium aminomethanesulfinate, zinc iminomethanesulfinate, sodium iminomethanesulfinate, zinc nitrilomethanesulfinate, sodium nitrilomethanesulfinate and aminoiminomethanesulfinic acid.

3. A composition as claimed in claim 2, which contains zinc dithionite and/or sodium dithionite.

4. A composition as claimed in claim 3, which contains sodium dithionite.

5. A composition as claimed in any one of claims 1 to 4, which contains, in total, from 15 to 90 % by weight, based on the total composition, of a sulfinic acid derivative or sulfinic acid derivatives.

6. A composition as claimed in any of claims 1 to 5, which contains from 0.01 to 10 % by weight, based on the total composition, of potassium carbonate.

## Revendications

1. Composition solide, contenant au moins un dérivé de l'acide sulfinique et du carbonate de potassium.

2. Composition selon la revendication 1, qui contient au moins un dérivé de l'acide sulfinique choisi dans le groupe constitué par le dithionite de zinc, le dithionite de sodium, le 1-hydroxyéthanesulfinate de zinc, le 1-hydroxyéthanesulfinate de sodium, le 1-aminoéthanesulfinate de zinc, le 1-aminoéthanesulfinate de sodium, le 1-iminoéthanesulfinate de zinc, le 1-iminoéthanesulfinate de sodium, le 1-nitriloéthanesulfinate de zinc, le 1-nitriloéthanesulfinate de sodium, l'hydroxyméthanesulfinate de zinc, l'hydroxyméthanesulfinate de sodium, l'aminométhanesulfinate de zinc, l'aminométhanesulfinate de sodium, l'iminométhanesulfinate de zinc, l'iminométhanesulfinate de sodium, le nitrilométhanesulfinate de zinc, le nitrilométhanesulfinate de sodium et l'acide aminoiminométhanesulfinique.

3. Composition selon la revendication 2, qui contient du dithionite de zinc et/ou du dithionite de sodium.

4. Composition selon la revendication 3, qui contient du dithionite de sodium.

5. Composition selon les revendications 1 à 4, qui contient au total 15 à 90% en poids de dérivé ou de dérivés de l'acide sulfinique par rapport à la composition totale.

6. Composition selon les revendications 1 à 5, qui contient 0,01 à 10% en poids de carbonate de potassium par rapport à la composition totale.
